# EUROPEAN PATENT SPECIFICATION

(11) **EP 2 640 347 B1**
(45) Date of publication and mention of the grant of the patent: **21.03.2018**
(21) Application number: 11791384.8
(22) Date of filing: 17.11.2011
(51) Int. Cl.: A61K 8/43, A61K 8/67, A61K 8/34, A61K 8/368, A61K 8/60, A61Q 19/00, A61Q 19/08, A61K 8/97

(54) **IMPROVEMENT OF THE APPEARANCE OF FINE LINES AND WRINKLES**
VERBESSERUNG DES ERSCHEINUNGSBILDES VON GESICHTSFALTEN UND GESICHTSFÄLTCHEN .
AMÉLIORATION DE L'ASPECT DES RIDES ET DES RIDULES DE LA PEAU DU VISAGE

(30) Priority: 19.11.2010 US 201061415752 P
(43) Date of publication of application: 25.09.2013
(73) Proprietor: The Procter & Gamble Company, Cincinnati, OH 45202 (US)
(72) Inventor: HAKOZAKI, Tomohiro, Cincinnati Ohio 45249 (US); LAUGHLIN, Leo, Timothy, II, Mason Ohio 45040 (US)
(74) Representative: Engisch, Gautier
(86) International application number: PCT/US2011/061167
(87) International publication number: WO 2012/068361

(56) References cited:
- WO-A1-2005/072696
- DE-A1- 19 928 112
- FR-A1- 2 849 374
- FR-A1- 2 913 598
- GB-A- 2 433 439
- US-A1- 2008 206 373
- US-B1- 6 174 533

## Description

### FIELD OF THE INVENTION

The present invention relates to improving the appearance of facial texture.

### BACKGROUND OF THE INVENTION

The epidermis, the outermost layer of the skin, comprises a cellular continuum of four layers: the stratum corneum, the granular layer, the spinous layer, and the basal layer. Each cellular layer in the epidermis represents various stages along a process in which basal epidermal keratinocytes undergo a continuous cycle of proliferation, differentiation, and apoptosis, moving upward from the basal layer to finally yield corneocytes. These corneocytes form the cornified layer known as the stratum corneum.

Basal keratinocytes reside at the lower portion of the epidermis. These mitotically active cells undergo a proliferative cycle to generate daughter cells that are physically dislocated upward into the spinous and granular layers and undergo the process of differentiation into corneocytes. On passing through the spinous and granular layers, the cells undergo morphological changes that render them flatter in structure as they lose their cellular viability, undergo alternate keratin expression profiles, and transform into cellular remnants. On average, a younger-aged epidermis turns over in about one month, shedding the older cells and replacing them with newer ones, but this process can increase to over forty days in older skin.

The stratum corneum's corneocytes remain connected to one other via proteins and lipids, creating a protective barrier between the organism and its outside environment. This tightly regulated epidermal permeability barrier functions as a physical and selective barrier against chemical and biological insults. Important functions of this barrier include attenuation of the penetration of free radicals and prevention of penetration of harmful radiation, including UV radiation, into deeper layers. The stratum corneum also acts as a permeability barrier and functions to prevent loss of body moisture to the outside environment. Dysfunction of this barrier can lead to chronic skin conditions, diseases, and in extreme cases can even threaten the viability of the organism.

Skin aging is a multifactorial process driven by both intrinsic (chronological aging) and extrinsic (environmental) factors, including ultraviolet (UV) exposure, environmental toxins, pollutants, and smoking. It is well known in the art that the ability of the stratum corneum to cyclically generate new layers of skin diminishes with age so that the stratum corneum turnover rate is substantially reduced in aged skin, with the cornified layer becoming gradually thinner.

This results in a reduction in the functioning capacity of the barrier so that harmful stimuli penetrate the stratum corneum more easily, leading to UV-damage, for example, of the underlying dermal layers, degradation of collagen and elastin, and eventually manifests in appearance as wrinkling and skin atrophy. Thinning of the stratum corneum by the sum of intrinsic and extrinsic aging factors increases the visible appearance of "macro-texture" (e.g., fine lines and wrinkles), and can also result in skin having a "microtexture" that is characterized by skin surface features that are smaller than those commonly referred to as fine lines and/or wrinkles. The present inventors recognized the desire for topically applied cosmetic compositions and associated methods of treatment that improve the appearance of textured facial skin, both macro-texture as well as micro-texture.

### SUMMARY OF THE INVENTION

The topical use of an effective amount of hexyldecanol to prevent, delay and/or reduce the appearance of fine lines and wrinkles.

### BRIEF DESCRIPTION OF THE DRAWING

It is believed that the present invention will be better understood from the following description taken in conjunction with the accompanying drawing.

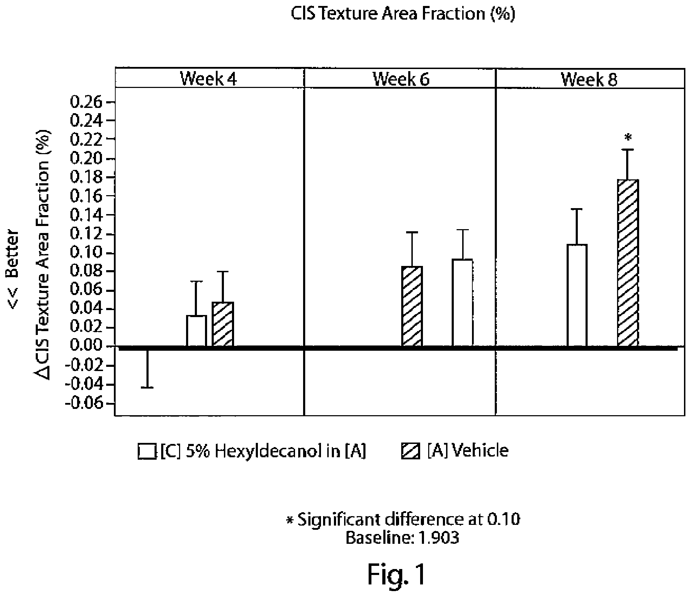

Figure 1 is a diagram showing the improvement in texture area fraction experienced by subjects in a facial texture test.

### DETAILED DESCRIPTION OF THE INVENTION

All percentages and ratios used herein are by weight of the total composition and all measurements made are at 25°C, unless otherwise designated.

The compositions in the present invention can comprise, consist essentially of, or consist of, the essential components as well as optional ingredients described herein. As used herein, "consisting essentially of" means that the composition or component may include additional ingredients, but only if the additional ingredients do not materially alter the basic and novel characteristics of the claimed compositions or methods.

The term "apply" or "application" as used in reference to a composition, means to topically apply or spread the compositions of the present invention onto an external human skin surface such as the epidermis.

The term "dermatologically acceptable" as used herein means that the compositions or components described are suitable for use in contact with human skin tissue without undue toxicity, incompatibility, instability, allergic response, and the like.

The term "effective amount" as used herein means an amount of a compound or composition sufficient to significantly induce a positive benefit.

The terms "texture" or "surface texture" when used in reference to human facial skin includes both macro-texture and micro-texture. Texture relates to the physical smoothness (evenness) and/or bumpiness (unevenness) of the skin's surface topography over a particular region. In some embodiments, "texture" refers to non-circular, non-elliptical, irregular, and/or elongated surface features and generally excludes pores and circular spots. For clarity, it should be understood that the regions of skin dimpling and nodularity commonly referred to as cellulite (which is caused by the herniation of subcutaneous fat within fibrous connective tissue) is not included within the meaning of "texture" as used herein.

The term "macro-texture" refers to facial skin surface features (e.g., depressions) typically referred to as fine lines and wrinkles that can be viewed with the naked human eye. Macro-texture skin surface features are larger in size than those which characterize micro-texture as used herein. Macro-texture generally excludes pores and other generally circular shapes.

The term "micro-texture" refers to small, irregular facial features (e.g., depressions) that are smaller in size than macro-texture as used herein. Micro-texture features are smaller in size than traditional fine lines and wrinkles, and generally exclude pores and other generally circular shapes. In some embodiments, micro-texture refers to elongate or irregular depressions less than 5 mm in length and thinner than 0.16 mm in breadth (i.e., features larger than those which are generally referred to as "fine lines and wrinkles" or "macro-texture").

The term "facial pores" or "pores" when used in reference to human facial skin refers generally to facial pores visible to the naked eye, although the term facial pores may also include pores that are not visible to the naked eye. A facial pore includes both the pore opening and the skin immediately adjacent to the opening that affects the visible appearance of the pore. In some instances, facial pores may have a pore area less than 2.0 mm², or 1.0 mm², or 0.1 mm², or less than 0.09 mm² or less than 0.08 mm² or less than 0.07 mm², or less than 0.05mm² and/or a pore area greater than 0.02 mm² or 0.04 mm². Facial pores generally, but not always, have a generally circular or elliptical shape at the skin surface.

The term "facial skin" as used herein refers to one or more of forehead, periorbital, cheek, perioral, chin, and nose skin surfaces.

The term "improving" when used in reference to facial skin texture includes preventing, delaying, and/or reducing the appearance of skin texture. "Improving" also thus includes increasing the smoothness of the skin surface topography. The degree of improvement can be measured quantitatively (e.g., by the Texture Area Fraction method set forth herein) and/or qualitatively (e.g., visual inspection).

### I. Compositions

In the present invention various compositions are used and, more specifically, compositions for topical application to the facial skin surface. The compositions may be in a wide variety of product forms that include, but are not limited to, solutions, suspensions, lotions, creams, gels, toners, sticks, pencil, sprays, aerosols, ointments, cleansing liquid washes and solid bars, shampoos and hair conditioners, pastes, foams, powders, mousses, shaving creams, wipes, strips, patches, electrically-powered patches, wound dressing and adhesive bandages, hydrogels, film-forming products, facial and skin masks (with and without insoluble sheet), make-up such as foundations, eye liners, and eye shadows, and the like. The composition form may follow from the particular dermatologically acceptable carrier chosen, if present in the composition.

### A. Hexyldecanol

Compositions comprise an effective amount of hexyldecanol. In particular embodiments, the composition may comprise from 1% to 10%, alternatively from 2.5% to 10%, alternatively from 2.5% to 6%, and alternatively from 4% to 6%, of hexyldecanol by weight of the total composition.

Hexyldecanol is the INCI name of the fatty alcohol also known as 2-hexyldecan-1-ol (IUPAC name), 2-hexyldecanol, or 2-Hexyl-1-decanol. (Chemical Formula: C₁₆H₃₄O, CAS number 2425-77-6). Hexyldecanol is a widely available cosmetic solvent and is commercially available from Sigma-Aldrich, Milwaukee, Wisconsin, USA.

### B. Optional Components

The compositions may contain a variety of other ingredients provided that they do not unacceptably alter the benefits of the invention. When present, compositions may contain from about 0.0001% to about 50%; from about 0.001% to about 20%; or, alternately, from about 0.01% to about 10%, by weight of the composition, of the optional components. The amounts listed herein are only to be used as a guide, as the optimum amount of the optional components used in a composition will depend on the specific active selected since their potency does vary considerably. Hence, the amount of some optional components useful in the present invention may be outside the ranges listed herein.

The optional components, when incorporated into the composition, should be suitable for use in contact with human skin tissue without undue toxicity, incompatibility, instability, allergic response, and the like. The compositions may include optional components such as anti-acne actives, desquamation actives, anti-cellulite agents, chelating agents, flavonoids, tanning active, non-vitamin antioxidants and radical scavengers, hair growth regulators, anti-wrinkle actives, anti-atrophy actives, minerals, phytosterols and/or plant hormones, N-acyl amino acid compounds, antimicrobial or antifungal actives, and other useful skin care actives, which are described in further detail in U.S. application publication No. US2006/0275237A1 and US2004/0175347A1.

The Personal Care Product Council's International Cosmetic Ingredient Dictionary and Handbook, Thirteenth Edition, describes a wide variety of non-limiting cosmetic and pharmaceutical ingredients commonly used in the skin care industry, which are suitable optional components for use in the compositions. Examples of these ingredient classes include: abrasives, absorbents, aesthetic components such as fragrances, pigments, colorings/colorants, essential oils, anti-caking agents, antifoaming agents, antimicrobials, binders, biological additives, buffering agents, bulking agents, chelating agents, chemical additives, colorants, cosmetic astringents, cosmetic biocides, denaturants, drug astringents, emollients, external analgesics, film formers or materials, opacifying agents, pH adjusters, preservatives, propellants, reducing agents, sequestrants, skin cooling agents, skin protectants, thickeners viscosity modifiers, vitamins, and combinations thereof.

Several classes of optional ingredients are discussed in more detail below.

### 1. Skin Tone Agents

In some embodiments, it may be desirable to also include a skin tone agent in the composition in combination with the hexyldecanol. As used herein, "skin tone" refers to generalized areas and/or regionalized areas (i.e. spots, age spots) of hyperpigmentation. As used herein, "improving the skin tone" means preventing or reducing the appearance of hyperpigmented areas.

The skin tone agents can be included to further improve overall skin tone. When present, the compositions contain up to about 50%, 40%, 30%, 20%, 10%, 5%, or 3%, by weight of the composition, of the skin tone agent. When present, the compositions contain at least about 0.001%, 0.01%, 0.1%, 0.2%, 0.5%, or 1%, by weight of the composition, of the skin tone agent. Suitable ranges include any combination of the lower and upper limits including suitable ranges from about 0.1% to about 50%; from about 0.2% to about 20%; or from about 1% to about 10%, by weight of the composition, of the skin tone agent. The amounts listed herein are only to be used as a guide, as the optimum amount of the skin tone agent will depend on the specific active selected since their potency does vary considerably.

Suitable skin tone agents include, but are not limited to, sugar amines, vitamin B3 compounds, arbutin, deoxyarbutin, 1,3-dihydroxy-4-alkylbenzene such as hexylresorcinol, ,bakuchoil (4-[(1E, 3S)-3-ethenyl-3,7-dimethyl - 1,6 octadienyl] phenol or monterpene phenol), pyrenoine (available from Biotech Marine, France), panicum miliaceum seed extract, arlatone dioic acid, cinnamic acid, ferulic acid, achromaxyl, methyl nicotinamide, oil soluble licorice extract, folic acid, undecylenic acid (i.e., undecenoic acid), zinc undecylenate, thiamine (Vitamin B1) and its hydrochloride, L-tryptophan, ficus benghalensis, phlorogine (laminaria) helianthus annuus (sunflower) and vitis vinifera (grape) leaf extract, carnosine (i.e., dragosine), methyl gentisate, 1,2-hexandiol and 1,2-octandiol (i.e., combination sold as Symdiol 68 by Symrise AG, Germany), inositol, decylenoylphenylalanine (e.g., sold under the tradename Sepiwhite by Seppic, France), kojic acid, hexamidine compounds, salicylic acid, and retinoids including retinol and retinyl propionate.

In certain embodiments, the additional skin tone agent is selected from vitamin B3 compounds, sugar amines, hexamidine compounds, salicylic acid, 1,3-dihydroxy-4-alkylbenzene such as hexylresorcinol, and retinoids. As used herein, "vitamin B₃ compound" means a compound having the formula: 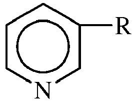 wherein R is - CONH₂ (*i.e*., niacinamide), - COOH (*i.e*., nicotinic acid) or - CH₂OH (*i.e*., nicotinyl alcohol); derivatives thereof; and salts of any of the foregoing. As used herein, "sugar amine" includes isomers and tautomers of such and its salts (*e.g*., HCl salt) and its derivatives. Examples of sugar amines include glucosamine, N-acetyl glucosamine, mannosamine, N-acetyl mannosamine, galactosamine, N-acetyl galactosamine, their isomers (*e.g*., stereoisomers), and their salts (*e.g*., HCl salt). As used herein, "hexaminide compound" means a compound having the formula: 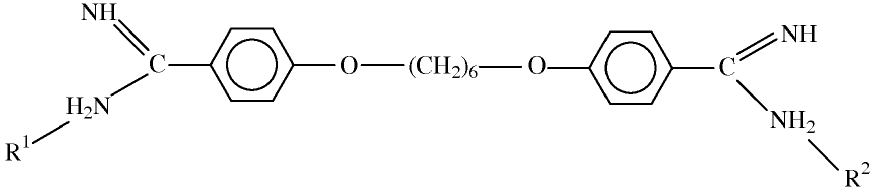 wherein R¹ and R² are optional or are organic acids (*e.g*., sulfonic acids, etc.). In one embodiment, hexamidine compound is hexamidine diisethionate.

### 2. Anti-Inflammatory Agents

The composition may additionally include an anti-inflammatory agent. When present, the compositions contain up to about 20%, 10%, 5%, 3%, or 1% by weight of the composition, of the anti-inflammatory agent. When present, the compositions contain at least about 0.001%, 0.01%, 0.1%, 0.2%, 0.3%, 0.5%, or 1%, by weight of the composition, of the anti-inflammatory agent. Suitable ranges include any combination of the lower and upper limits. Suitable anti-inflammatory agents include, but are not limited to nonsteroidal anti-inflammatory agents (NSAIDS including but not limited to ibuprofen, naproxen, flufenamic acid, etofenamate, aspirin, mefenamic acid, meclofenamic acid, piroxicam and felbinac), glycyrrhizic acid (also known as glycyrrhizin, glycyrrhixinic acid, and glycyrrhetinic acid glycoside) and salts such as dipotassium glycyrrhizate, glycyrrhetenic acid, licorice extracts, bisabolol (*e.g*., alpha bisabolol), manjistha (extracted from plants in the genus *Rubia*, particularly *Rubia cordifolia*), and guggal (extracted from plants in the genus *Commiphora*, particularly *Commiphora mukul*), kola extract, chamomile, red clover extract, and sea whip extract (extracts from plant in the order *Gorgonacea*), derivatives of any of the foregoing, and mixtures thereof.

### 3. Sunscreen Actives

The compositions may comprise one or more sunscreen actives (or sunscreen agents) and/or ultraviolet light absorbers. Herein, "sunscreen active" collectively includes, sunscreen actives, sunscreen agents, and/or ultraviolet light absorbers. Sunscreen actives include both sunscreen agents and physical sunblocks. Sunscreen actives may be organic or inorganic. Examples of suitable sunscreen actives are disclosed in Personal Care Product Council's International Cosmetic Ingredient Dictionary and Handbook, Thirteenth Edition, as "sunscreen agents." Particularly suitable sunscreen actives are 2-ethylhexyl-p-methoxycinnamate (commercially available as PARSOL™ MCX), 4,4'-t-butyl methoxydibenzoyl-methane (commercially available as PARSOL™ 1789), 2-hydroxy-4-methoxybenzophenone, octyldimethyl-p-aminobenzoic acid, digalloyltrioleate, 2,2-dihydroxy-4-methoxybenzophenone, ethyl-4-(bis(hydroxypropyl))aminobenzoate, 2-ethylhexyl-2-cyano-3,3-diphenylacrylate, 2-ethylhexyl-salicylate, glyceryl-p-aminobenzoate, 3,3,5-tri-methylcyclohexylsalicylate, menthyl anthranilate, p-dimethyl-aminobenzoic acid or aminobenzoate, 2-ethylhexyl-p-dimethyl-aminobenzoate, 2-phenylbenzimidazole-5-sulfonic acid, 2-(p-dimethylaminophenyl)-5-sulfonicbenzoxazoic acid, octocrylene, zinc oxide, benzylidene camphor and derivatives thereof, titanium dioxide, and mixtures thereof.

In one embodiment, the composition may comprise from about 1% to about 20%, and alternatively from about 2% to about 10% by weight of the composition, of the sunscreen active. Exact amounts will vary depending upon the chosen sunscreen active and the desired Sun Protection Factor (SPF), which is within the knowledge of one of skilled in the art.

### C. Dermatologically Acceptable Carrier

The compositions may also comprise a dermatologically acceptable carrier (which may be referred to as "carrier") for the composition. The phrase "dermatologically acceptable carrier", as used herein, means that the carrier is suitable for topical application to the skin, has good aesthetic properties, is compatible with the actives in the composition, and will not cause any unreasonable safety or toxicity concerns. In one embodiment, the carrier is present at a level of from about 50% to about 99%, about 60% to about 98%, about 70% to about 98%, or, alternatively, from about 80% to about 95%, by weight of the composition.

The carrier can be in a wide variety of forms. Non-limiting examples include simple solutions (*e.g.,* aqueous, organic solvent, or oil based), emulsions, and solid forms (*e.g.,* gels, sticks, flowable solids, or amorphous materials). In certain embodiments, the dermatologically acceptable carrier is in the form of an emulsion. Emulsion may be generally classified as having a continuous aqueous phase (*e.g*., oil-in-water and water-in-oil-in-water) or a continuous oil phase (*e.g*., water-in-oil and oil-in-water-in-oil). The oil phase may comprise silicone oils, non-silicone oils such as hydrocarbon oils, esters, ethers, and the like, and mixtures thereof.

The aqueous phase typically comprises water. However, in other embodiments, the aqueous phase may comprise components other than water, including but not limited to watersoluble moisturizing agents, conditioning agents, anti-microbials, humectants and/or other watersoluble skin care actives. In one embodiment, the non-water component of the composition comprises a humectant such as glycerin and/or other polyols. However, it should be recognized that the composition may be substantially (*i.e*., less than 1% water) or fully anhydrous.

A suitable carrier is selected to yield a desired product form. Furthermore, the solubility or dispersibility of the components (e.g., hexyldecanol, sunscreen active, additional components) may dictate the form and character of the carrier. In one embodiment, an oil-in-water or water-in-oil emulsion is preferred.

Emulsions may further comprise an emulsifier. The composition may comprise any suitable percentage of emulsifier to sufficiently emulsify the carrier. Suitable weight ranges include from about 0.1% to about 10% or about 0.2% to about 5% of an emulsifier, based on the weight of the composition. Emulsifiers may be nonionic, anionic or cationic. Suitable emulsifiers are disclosed in, for example, U.S. Patent 3,755,560, U.S. Patent 4,421,769, and McCutcheon's Detergents and Emulsifiers, North American Edition, pages 317-324 (1986). Suitable emulsions may have a wide range of viscosities, depending on the desired product form.

The carrier may further comprise a thickening agent as are well known in the art to provide compositions having a suitable viscosity and rheological character.

### II. Exemplary Compositions

Table 1 sets forth non-limiting examples of the compositions.

All Examples may be used to improve the appearance of one or more areas of facial texture.

**Table 1**

| **Component** | **Ex. A** | **Ex. B** | **Ex. C** | **Ex. D** | **Ex. E** |
|---|---|---|---|---|---|
| Hexyldecanol *1 | 5.000 | 4.000 | 5.000 | 3.000 | 6.000 |
| N-Acetylglucosamine | 0 | 0 | 2.000 | 0 | 0 |
| Hexamidine Diisethionate | 0 | | | 0.090 | 0.090 |
| Undecylenoyl-phenylalanine *2 (neutralized) | 0 | 1.000 | 0.500 | 0 | 0 |
| Dipotassium Glycyrrhizate | 0 | 0.300 | 0.100 | 0.100 | 0.100 |
| Niacinamide | 5.000 | 5.000 | 5.000 | 5.000 | 5.000 |
| Isohexadecane | 3.000 | 3.000 | 3.000 | 3.000 | 3.000 |
| Isopropyl isostearate | 1.330 | 1.330 | 1.330 | 1.330 | 1.330 |
| Cetearyl glucoside + cetearyl alcohol *3 | 0.200 | 0.200 | 0.200 | 0.200 | 0.200 |
| Behenyl alcohol | 0.400 | 0.400 | 0.400 | 0.400 | 0.400 |
| Cetyl alcohol | 0.320 | 0.320 | 0.320 | 0.320 | 0.320 |
| Stearyl alcohol | 0.480 | 0.480 | 0.480 | 0.480 | 0.480 |
| Tocopheryl acetate | 0.500 | 0.500 | 0.500 | 0.500 | 0.500 |
| PEG-100 stearate | 0.100 | 0.100 | 0.100 | 0.100 | 0.100 |
| Glycerin | 7.000 | 7.000 | 7.000 | 7.000 | 7.000 |
| Polyacrylamide + C13-14 isoparaffin + laureth-7 *4 | 2.000 | 2.000 | 2.000 | 2.000 | 2.000 |
| Disodium EDTA | 0.100 | 0.100 | 0.100 | 0.100 | 0.100 |
| Benzyl alcohol | 0.400 | 0.400 | 0.400 | 0.400 | 0.400 |
| Dimethicone/ Dimethiconol *5 | 2.000 | 2.000 | 2.000 | 2.000 | 2.000 |
| Homosalate | 0 | 0 | 0 | 0 | 9.000 |
| Avobenzone | 0 | 0 | 0 | 0 | 3.000 |
| Octocrylene | 0 | 0 | 0 | 0 | 2.600 |
| Oxybenzone | 0 | 0 | 0 | 0 | 1.000 |
| Octisalate | 0 | 0 | 0 | 0 | 4.500 |
| Water | QS | QS | QS | QS | QS |
| TOTAL | 100 | 100 | 100 | 100 | 100 |

| | | | | | |
|---|---|---|---|---|---|
| *1 - Hexyldecanol available from Sigma-Aldrich, USA. *2 - Sepiwhite available from SEPPIC, France. *3 - Emulgade PL 68/50 available from Cognis GmbH. *4 - Sepigel 305, available from SEPPIC, France. *5 - Dow Corning DC1503 available from Dow Corning, Inc., Midland, MI. | | | | | |

The compositions are generally prepared by conventional methods such as are known in the art of making topical compositions. Such methods typically involve mixing of the ingredients in one or more steps to a relatively uniform state, with or without heating, cooling, application of vacuum, and the like. Typically, emulsions are prepared by first mixing the aqueous phase materials separately from the fatty phase materials and then combining the two phases as appropriate to yield the desired continuous phase. The compositions are preferably prepared such as to optimize stability (physical stability, chemical stability, photostability) and/or delivery of the active materials. This optimization may include appropriate pH (e.g., less than 7), exclusion of materials that can complex with the active agent and thus negatively impact stability or delivery (e.g., exclusion of contaminating iron), use of approaches to prevent complex formation (e.g., appropriate dispersing agents or dual compartment packaging), use of appropriate photostability approaches (e.g., incorporation of sunscreen/sunblock, use of opaque packaging), etc.

### III. Methods of Treatment

Various methods of treatment, application, regulation, or improvement may utilize the aforementioned compositions. In one embodiment, the method includes the step of identifying facial texture for improvement by the composition. The facial texture may be identified by the user or a third party such as a dermatologist, cosmetician, or other caregiver. Identification may be done by visual inspection of the skin for facial texture in need of treatment based on appearance. Identification may also be done by commercially available imaging devices such the VISIA® Complexion Analysis system (available from Canfield Scientific, Inc., Fairfield, NJ). The device is capable of collecting images of the skin and identifying facial textures. In some instances, the method comprises the step of identifying a plurality of facial texture areas for treatment by the composition. Identification of the facial texture may occur on any facial skin surface, including the forehead, perioral, chin, periorbital, nose, and/or cheek skin surfaces.

The method may comprise the step of applying the composition to facial texture, which may have been previously identified. Many regimens exist for the application of the composition to the facial texture. The composition may be applied at least once a day, twice a day, or on a more frequent daily basis, during a treatment period. When applied twice daily, the first and second applications are separated by at least 1 to about 12 hours. Typically, the composition may be applied in the morning and/or in the evening before bed.

The treatment period is ideally of sufficient time to provide an improvement in the appearance of the facial texture. The treatment period may be at least about 1 week. The treatment period may last about 4 weeks or about 8 weeks. In certain embodiments, the treatment period will extend over multiple months (*i.e*., 3-12 months) or multiple years. In one embodiment the composition is applied to the facial texture at least once a day during a treatment period of at least about 4 weeks or at least about 8 weeks. In one embodiment the composition is applied to the facial texture twice a day during a treatment period of at least about 4 weeks or 8 weeks.

The step of applying the composition to the facial texture may be done by localized application. In reference to application of the composition, the term "localized", "local", or "locally" mean that the composition is delivered to the targeted area (such as the region of facial texture) while minimizing delivery to skin surface not requiring treatment. The composition may be applied and lightly massaged into the facial texture. It is recognized that localized application does allow for a reasonable amount of the composition to be applied to areas adjacent the facial texture (*i.e*., the composition is unlikely to be applied or to remain within the boundary of the facial texture without some spreading). The form of the composition or the dermatologically acceptable carrier should be selected to facilitate localized application. While certain embodiments of the present invention contemplate applying a composition locally to facial texture, it will be appreciated that compositions can be applied more generally or broadly to one or more facial skin surfaces to reduce the appearance of facial texture within those facial skin regions.

In some embodiments, the composition may be delivered by a variety of applicators appropriate for localized and general application. In another embodiment, the composition is applied to the one or more facial texture regions and more generally to one or more facial skin surfaces contemporaneously (*i.e*., over a period of less than 30 minutes or, more typically, less than 5 minutes). While some methods described herein contemplate applying the compositions with an applicator, it will be appreciated that applicators are not required and the compositions can also be applied directly by using one's finger or in other conventional manners.

For general application to a skin surface and, particularly a facial skin surface, the dosed amount of the composition may be between about 1 to about 50 uL/cm² per application (*i.e*., per single application to the skin surfaces).

One suitable method of improving the appearance of facial texture includes the step of topically applying a composition comprising an effective amount of hexyldecanol to the facial texture on a skin surface, wherein the composition is applied for a period of time sufficient for hexyldecanol to improve the appearance of the facial texture. Another suitable method of improving the appearance of facial textures includes the steps of identifying facial texture on a skin surface, applying a composition comprising an effective amount of hexyldecanol to the facial texture on the skin surface, wherein the composition is applied for a period of time sufficient for hexyldecanol to improve the appearance of the facial texture.

### VI. Mechanisms of Action

As discussed above, the stratum corneum is a tightly regulated epidermal permeability barrier and functions as a physical and selective barrier against chemical and biological insults, as well as acts as a permeability barrier to prevent loss of body moisture to the outside environment. The stratum corneum's cells control these barriers by regulating the movement of water, ions, and proteins across them. The health and resulting cosmetic appearance of the facial skin is closely tied to the skin's barrier function and permeability.

The inventors herein have found that stimulating hyaluronic acid ("HA") production can improve the appearance of facial texture. HA is known to affect the skin's moisture level and acts as a moisture sponge, binding up to 1000 times it's own weight in water, however the enzymatic steps that constitute extracellular and intracellular HA cycles are not yet fully understood. HA production can help "firm up" the skin surface by maintaining good moisturization levels. Accordingly, the present inventors have also found that applying an effective amount of a material that regulates HA production can also improve the appearance of facial texture. In some embodiments, hexyldecanol is used as the material for regulating HA production.

According to some embodiments, the method of improving the appearance of facial skin texture comprises the step of topically applying a composition comprising an effective amount of a material that regulates Hyaluronic Acid (HA) production to a region of facial skin, wherein the composition is applied for a period of time sufficient for said material to improve the appearance of the facial texture.

In other embodiments, the method of improving the appearance of facial skin texture comprises the steps of (a) identifying a region of texture skin on a facial skin surface, and (b) applying a composition comprising an effective amount of a material that regulates Hyaluronic Acid (HA) production to the region of texture facial skin on the facial skin surface, wherein the composition is applied for a period of time sufficient for said material to improve the appearance of the facial texture.

### V. In Vivo Testing for Facial Texture

A 9 week *in vivo* study was conducted using a round robin, vehicle controlled, split face design including a 1 week normalization period with 330 subjects.

Treatment Regimen - The regimen begins with a one week washout period. Each morning the subject is to wash her face with a suitable cleanser (*e.g*., Olay Natural Science Deep Purify Cleanser, available from The Procter & Gamble Company, Cincinnati, OH), gently dry with a towel, apply a stock moisturizer (*e.g*., Vehicle as described in Table 2 with 3% glycerine, no panthenol, and 0.3% disodium EDTA) to the appropriate side of the face, wait 5 minutes, and then apply a UV blocking lotion (*e.g*., Olay Complete All Day Moisturizing Lotion SPF 15, available from The Procter & Gamble Company, Cincinnati, OH). Each night the subject is to wash her face with a suitable cleanser (*e.g.,* Olay Natural Science Deep Purify Cleanser, available from The Procter & Gamble Company, Cincinnati, OH), gently dry with a towel, and apply the stock moisturizer.

Each subject receives two coded test formulations for twice daily application to either the left or right side of the face. Each morning the subject is to wash her face with a suitable cleanser (*e.g*., Olay Natural Science Deep Purify Cleanser, available from The Procter & Gamble Company, Cincinnati, OH), gently dry with a towel, apply the test formulation to the appropriate side of the face, wait 5 minutes, and then apply a UV blocking lotion (*e.g*., Olay Complete All Day Moisturizing Lotion SPF 15, available from The Procter & Gamble Company, Cincinnati, OH). Each night the subject is to wash her face with a suitable cleanser (*e.g.,* Olay Natural Science Deep Purify Cleanser, available from The Procter & Gamble Company, Cincinnati, OH), gently dry with a towel, and apply the test formulation to the appropriate side of the face. Participants are to apply 0.5g of the appropriate test formulation on each side of the face. The test formulation should be applied with the fingers using gentle pressure and in a circular motion. Test formulations included a vehicle control, and the vehicle + 5% Hexyldecanol. These test formulas are set forth in Table 2.

**Table 2**

| Component | Vehicle | Vehicle + 5% Hexyldecanol |
|---|---|---|
| Water | Q.S. | Q.S. |
| Hexyldecanol *A | --- | 5.000 |
| Niacinamide | --- | --- |
| Glycerin | 7.0000 | 7.0000 |
| Isohexadecane | 3.0000 | 3.0000 |
| Polyacrylamide(and)C 13-14 Isoparaffin(and)Laureth-7 *B | 2.0000 | 2.0000 |
| Dimethicone and Dimethiconol *C | 2.0000 | 2.0000 |
| Isopropyl Isostearate | 1.3300 | 1.3300 |
| Tocopheryl Acetate | 0.5000 | 0.5000 |
| Panthenol | 1.0000 | 1.0000 |
| Cetyl Alcohol | 0.3200 | 0.3200 |
| Sucrose Polycottonseedate | 0.6700 | 0.6700 |
| Cetearyl Glucoside/Cetearyl Alcohol *D | 0.2000 | 0.2000 |
| Stearyl Alcohol | 0.4800 | 0.4800 |
| Behenyl Alcohol | 0.4000 | 0.4000 |
| Polymethylsilsesquioxane *E | 0.2500 | 0.2500 |
| Ethylparaben | 0.2000 | 0.2000 |
| Propylparaben | 0.1000 | 0.1000 |
| Disodium EDTA | 0.1000 | 0.1000 |
| Benzyl Alcohol | 0.2500 | 0.2500 |
| PEG-100 Stearate | 0.1000 | 0.1000 |

| | | |
|---|---|---|
| *A - Hexyldecanol available from Sigma-Aldrich, USA or Cognis, Germany. *B - Sepigel 305, available from SEPPIC, France. *C - Dow Corning 1503 Fluid, available from Dow Corning, Midland, MI. *D - Emulglade PL 68/50, available from Cognis GmbH, Germany. *E - Tospearl 2000, available from Momentive Performance Materials, Albany, NY. | | |

Images of the facial treatment sites are captured at baseline (week 0), and after 4 and 8 weeks of treatment and analyzed for changes to facial texture. Prior to image collection the participant's face is washed with the above referenced cleanser and allowed to dry (approximately 20 minutes). Images are collected of the right and left side of the participant's face. Images are collected using a digital camera (*e.g*., Fuji F2 Pro digital SLR) equipped with a suitable lens for facial imaging (*e.g*., 60mm Nikor lens), mounted in a standardised illumination box fitted with head-positioning. The camera was calibrated daily using a GretagMacbeth neutral 8.0 grey colour board in front of the camera. Left and right views of the face were standardised-that is, the same focal distance from the camera lens to the face, same magnification, same head position so that the camera angle was the same relative to the face surface, and exactly the same lighting. Images are saved in a suitable file format such as RAW format at a suitable camera resolution. Lighting is provided by a flash source (*e.g.,* 1000W strobe with color temperature of about 5600K). The camera and lighting are equipped with polarizing filters to reduce specular reflection.

The region of interest (ROI), in this case the upper cheek area, was marked manually based on 12 predefined facial landmarks around the cheek-for example, corners of the eye, bridge of the nose, corners of the mouth. The degree of textured skin in the ROI were quantified objectively using image analysis algorithms based on an Optimus software platform, which automatically locates each surface feature and quantifies the total number, length and area of facial features shorter than 5 mm and less than 0.16 mm wide, known magnification used to convert pixel data to actual length and area data. Thresholds were based on "clinically important" facial texture-that is, excluding lines greater than 5 mm and broader than 0.16 mm, which fall under the heading of "fine lines and wrinkles".

Because the ROI varies in shape and size, total textured area was normalised to total ROI size to yield a Texture Area Fraction (TAF)-that is, fractional ROI area occupied by facial texture. Group statistical analysis used the mean TAF on the left and right sides of the face for each subject. Stata 8.1 (Stata Corp, Lakeway Drive College Station, Texas, USA) was used for the statistical analysis. A multivariate logistic regression analysis obtained the maximum likelihood OR estimates and corresponding 95% CIs. Data collected from the image are used to calculate Texture Area Fraction, which is an indication of the level of texture present. A lower Texture Area Fraction reflects a smoother texture.

Hexyldecanol performed best at 8 weeks, significantly (p <= 0.10) reducing Texture Area Fraction better than the control. Figure 1 summarizes these results. This test was performed in Beijing, China.

### VI. Test Methods

The following methods are provided to illustrate the invention.

### A. Hyaluronic Acid (HA) Synthase Expression

### 1. Keratinocyte Culture:

Individual experiments (referred to as batches) are generally comprised of 60 Affymetrix GeneChips® (referred to as "instances") containing 6 vehicle control samples, 2 positive control samples and 52 individual test material samples. Duplication of test materials is performed across batches. In vitro testing is performed in 6-well plates to provide sufficient RNA for GeneChip® analysis (2-4 ug total RNA yield/well). Human telomerized keratinocytes are grown in EpiLife® media with 1X Human Keratinocyte Growth Supplement (Invitrogen) on collagen I coated cell culture flasks and plates (Becton Dickinson). Cells are seeded into 6-well plates at 20,000 cells /cm2 24 hours before chemical exposure. At t= - 24 hours cells are trypsinized from T-75 flasks and plated into 6-well plates in basal growth medium. At t=0 media is removed and replaced with the appropriate dosing solution as per the experimental design. Dosing solutions are prepared the previous day in sterile 4 ml Falcon snap cap tubes. After 6 hours of chemical exposure cells are viewed and imaged. Cells are then lysed with 350ul/well of RLT buffer containing β-mercaptoethanol (Qiagen), transferred to a 96-well plate, and stored at - 20° C.

### 2. Transcriptional Screening - RNA analyzed with gene chips

Total RNA Isolation: Cells suspended in ∼350 ul of RNEasy RLT Buffer (QIAgen, Hilden, Germany) plus beta-mercaptoethanol and 400 ul of Agencourt RNAClean paramagnetic beads (Beckman Coulter Genomics, Danvers, MA). RNA was purified using a modification of the RNEasy procedure that has been optimized for automation on the Affymetrix (Santa Clara, CA) GCAS instrument.

GeneChip Target Synthesis and GeneChip Processing: 1 ug of purified total RNA is converted to cRNA GeneChip target using the Ambion (Austin, TX) MessageAmp II kit and protocol provided. 20 ug of cRNA target were fragmented and hybridized to Affymetrix U133plus2.0 arrays. Hybridization, washing, and scanning procedures were carried out according to the Affymetrix Expression Analysis protocol. Complete protocols for target synthesis and GeneChip processing can be found on Affymetrix website which is currently (11/15/2010) (www.affymetrix.com). The referenced manual is P/N 702232 Revision 3 copyright 2005 - 2009.

GeneChip Analysis: GeneChip scans were converted to tabular data using the Affymetrix MAS5 algorithm, which is also described and found at the website for Affymetrix (www.affymetrix.com).

Data quality was determined using a variety of statistical measures, including t-tests, scatter biplots, and principal components analysis, depending upon the source and character of the data.

Data Analysis: Affymetrix probe sets are rank ordered according to p-values, and probes showing changes with p-values > 0.1 are excluded from the analysis as these are deemed not significant. Probe sets are coupled to gene annotation data by batch query of Affymetrix database. Visual inspection of resulting transcription changes with annotation is used to find genes of interest. For facial texture, transcripts for Hyaluronic acid synthase 2 were chosen.

Results: Using generally the methods described above, a microarray analysis of six Affymetrix GeneChips was processed for a keratinocyte cell culture dosed with a 10 micro molar concentration of hexyldecanol. The transcriptional expression level for the probe set IDs associated with the HAS-2 gene (Hyaluronic Acid Synthase-2 gene) had an average fold change of 1.336 (p-value = 0.0366).

## Claims

1. The topical use of an effective amount of hexyldecanol to prevent, delay and/or reduce the appearance of fine lines and wrinkles.

2. Use according to claim 1 in that hexyldecanol stimulates hyaluronic acid (HA) production.

3. Use according to any of the preceding claims, wherein hexyldecanol is further combined with a skin tone agent, preferably selected from the group consisting of vitamin B3 compounds, sugar amines, hexamidine compounds, salicylic acid, 1,3-dihydroxy-4-alkylbenzene, retinoids, and combinations thereof.

4. Use according to any of the preceding claims, wherein hexyldecanol is applied to facial pores region at a level of from 1 to 50 uL/cm².

## Patentansprüche

1. Topische Anwendung einer wirksamen Menge Hexyldecanol zur Prävention, Verzögerung und/oder Reduzierung von Fältchen- und Faltenbildung.

2. Anwendung nach Anspruch 1, wobei Hexyldecanol die Produktion von Hyaluronsäure (HS) anregt.

3. Anwendung nach einem der vorstehenden Ansprüche, wobei Hexyldecanol ferner mit einem Hauttonisierungsmittel kombiniert wird, vorzugsweise ausgewählt aus der Gruppe bestehend aus Vitamin B3-Verbindungen, Zucker-Aminen, Hexamidin-Verbindungen, Salicylsäure, 1,3-Dihydroxy-4-Alkylbenzol, Retinoiden und Kombinationen davon.

4. Anwendung nach einem der vorstehenden Ansprüche, wobei Hexyldecanol auf den Gesichtsporenbereich in einer Menge von 1 bis 50 ul/cm² aufgetragen wird.

## Revendications

1. Utilisation topique d'une quantité efficace d'hexyldécanol pour empêcher, retarder et/ou réduire l'apparition de rides et ridules.

2. Utilisation selon la revendication 1, **caractérisée en ce que** l'hexyldécanol stimule la production d'acide hyaluronique (HA).

3. Utilisation selon l'une quelconque des revendications précédentes, dans laquelle l'hexyldécanol est en outre combiné avec un agent de teint de la peau, choisi de préférence dans le groupe constitué de composés de vitamine B3, amines de sucre, composés hexamidine, acide salicylique, 1,3-dihydroxy-4-alkylbenzène, rétinoïdes et leurs combinaisons.

4. Utilisation selon l'une quelconque des revendications précédentes, dans laquelle l'hexyldécanol est appliqué sur des régions de pores du visage à un taux allant de 1 à 50 uL/cm².
